# EUROPÄISCHE PATENTSCHRIFT

(11) **EP 2 705 062 B1**
(45) Veröffentlichungstag und Bekanntmachung des Hinweises auf die Patenterteilung: **23.03.2016**
(21) Anmeldenummer: 12719670.7
(22) Anmeldetag: 02.05.2012
(51) Int. Cl.: C08F 220/36, C08F 220/38, A61K 8/81, A61K 47/32, A61Q 5/00, A61Q 19/00, C09K 8/24

(54) **SCHERSTABILE POLYMERSYSTEME, DEREN HERSTELLUNG SOWIE DEREN VERWENDUNG ALS VERDICKER**
SHEAR STABLE POLYMERS, THEIR PREPARATION AND THEIR USE AS THICKENER
POLYMÈRES STABLES AUX CISAILLEMENT, LEURS PROCÉDÉ DE PRÉPARATION ET LEURS UTILISATIONS EN TANT QUE ÉPAISSISSANT

(30) Priorität: 04.05.2011 DE 102011100430
(43) Veröffentlichungstag der Anmeldung: 12.03.2014
(73) Patentinhaber: Rhodia Operations, 93306 Aubervilliers (FR)
(72) Erfinder: LERCH, Alexander, 63571 Gelnhausen (DE); SCHADE, Manfred, 2596 JG Den Haag (NL); BURKHART, Alexander, 45239 Essen (DE); RITTER, Helmut, 42111 Wuppertal (DE)
(74) Vertreter: Ter Meer Steinmeister & Partner
(86) Internationale Anmeldenummer: PCT/EP2012/058029
(87) Internationale Veröffentlichungsnummer: WO 2012/150262

(56) Entgegenhaltungen:
- EP-A1- 0 318 612
- WO-A1-93/22358
- DE-A1- 10 000 648
- US-A- 5 080 809
- US-A- 5 311 223
- US-A1- 2004 024 154

## Beschreibung

Gegenstand der vorliegenden Erfindung ist die Bereitstellung von neuen hochverdickenden Polymeren und deren Anwendung in wässrigen Formulierungen, die sich durch erhöhte Scherstabilität auszeichnen.

### Beschreibung

Polyelektrolyte gehören mit ihren viskositätserhöhenden Eigenschaften zu den essentiellen Bestandteilen von kosmetischen und pharmazeutischen Zubereitungen. Zur Einstellung anwendungsorientierter rheologischer Eigenschaften ist die Verwendung von geeigneten wasserlöslichen oder wasserquellbaren Polymeren erforderlich.

Wurden in der Vergangenheit hauptsächlich natürlich vorkommende, auf Polysacchariden basierende Systeme wie GuarGum oder modifizierte Cellulosen (CMC, HEC, CMHEC etc.) eingesetzt, traten zum Ende des letzten Jahrhunderts die synthetischen polymeren Verdicker immer mehr in den Vordergrund. Ihr chemisch kontrollierbarer Aufbau macht sie den natürlichen oder teilsynthetischen Systemen mit ihren großen Qualitätsschwankungen in vielen Anwendungsbereichen überlegen. Im Mittelpunkt standen dabei hochmolekulare vernetzte Polyacrylsäuren, deren breites Einsatzspektrum sie zu den auch heute noch am häufigsten verwendeten Polyelektolyt-Verdickern macht. Patente aus den 80er Jahren des letzten Jahrhunderts, vornehmlich von BF-Goodrich, beschreiben die Entwicklung der reinen Acrylatverdicker bis hin zu den modernen assoziativen Systemen von BF-Goodrich bzw. Rohm und Haas.

Die anionischen Verdickungssysteme entstehen dadurch, dass ihre ursprünglich sauren Monomereinheiten durch Abgabe eines Protons an eine geeignete Base neutralisiert werden. Die resultierende Salzstruktur erlaubt in wässriger Umgebung eine partielle räumliche Trennung der Ionenpaare. Dadurch findet eine elektrostatische Abstoßung der an der polymeren Hauptkette fixierten anionischen Wiederholungseinheiten statt, wodurch ein makroskopisches "Aufquellen" des Systems in wässriger Phase resultiert.

Je nach chemischer Natur der verwendeten Monomereinheiten begrenzt der pKₛ-Wert der entsprechenden polymeren Säure die Lösungseigenschaften der Polymere in Wasser. Bei Anwendung von verdickenden Systemen, die auf (Meth)acrylsäure aufbauen, ist in wässriger Lösung die Polyelektrolytbildung auf pH Werte oberhalb von ca 5 beschränkt. Zufriedenstellende Verdickungseffekte unterhalb dieses pH-Wertes können nur durch die Neutralisation von stärkeren Säuren, wie Polyphosphorsäuren oder Polysulfonsäuren erreicht werden. Als Beispiel hierfür ist das Seppigel® (Polyacrylamid/Polyacrylamidoproypylmethylensulfonat) der Firma Seppic zu nennen. Weiterhin beschreibt DE10000648 A1 anionische Polymere auf Basis AMPS und Copolymere aus AMPS mit neutralen Monomeren, wie z.B. NVP und deren Einsatz als Verdickersysteme

Synthetische polymere Verdicker mit ihren steuerbaren Eigenschaften erlauben die Formulierung neuer Zubereitungen. Zusätzlich zu der in hohem Masse bedeutsamen Verwendung von kovalent vernetzten Polyelektrolyten als "Superabsorber" in Hygieneartikeln (wie z.B. Babywindeln) gibt es weitere sehr unterschiedliche Anwendungsgebiete, wie Dispersionsstabilisatoren, Flokkulantien (z.B. für die Abwasserreinigung), sowie als Trennmittel in Gemischen. Weitere Anwendungen sind in silikathaltigen Systemen wie Bohrschlämmen oder bei der Aufarbeitung von Böden zu finden. Auch erhalten Lacke, Druckpasten oder - tinten ihr essentielles Eigenschaftsprofil durch geeignete Polyelektrolyt-Verdicker.

Elektrostatische Wechselwirkungen mit polaren Strukturen ist die Ursache für die Wirkungsweise der polymeren Salze. Die Kontrolle der entsprechenden Wechselwirkungsenergie stellt eine Herausforderung zur Variation und Verbesserung der Eigenschaften dar. Rein anionische bzw. kationische Polymer-Systeme werden bereits seit Jahrzehnten mit Erfolg eingesetzt. Die anwendungstechnischen Grenzen jeder der beiden Substanzgruppen werden allerdings dann erreicht, wenn Mischungen beider Klassen eingesetzt werden. In wässrigen oder organischen Lösungen neutralisieren sich die jeweils gegenpoligen Ladungen und führen oft zum Ausfällen aus der Lösung unter Entropiegewinn, da die jeweils kleinen Gegenionen kombinieren und dynamisch mobile niedermolekulare Salze bilden. Dieser Umstand verhindert bei vielen interessanten Anwendungen den gleichzeitigen Einsatz von anionischen und kationischen Strukturen und macht diese damit für viele Bereiche unzugänglich. Es wurde oft vergeblich versucht, durch chemische Kombination von unterschiedlich geladenen Monomeren zu entsprechenden Polyanion-Polykation Strukturen mit den gewünschten Eigenschaften zu kommen. Bereits während der Polymersynthese treten oft unüberwindliche Schwierigkeiten auf, die naturgemäß auf die unterschiedlichen, sich gegenseitig neutralisierenden Ladungen, zurückgeführt werden können.

Es gibt jedoch Monomerstrukturen, die beide Ladungsarten bereits in sich vereinen. Diese so genannten polymerisierbaren Betaine tragen in einer Monomereinheit sowohl eine negative als auch eine positive Ladung, die sich aufgrund ihrer räumlichen Nähe vollkommen selbst neutralisieren. Ein einfaches Gegenion ist aufgrund der Gesamtneutralität nicht existent. Polymere Betaine sind seit langem für ihre speziellen Dispergiereigenschaften bekannt, da sie nach außen hin zwar ungeladen sind, aber dennoch eine ungleiche Verteilung der Ladungsdichte im Molekül aufweisen. Diese Eigenschaft befähigt sie, zum Beispiel auf alle polaren Oberflächen aufzuziehen. Das fehlende frei bewegliche Gegenion lässt betainische Struktureinheiten auch im mikroskopischen Bereich nach außen hin neutral erscheinen. Betainische Systeme wurden bislang eingesetzt um in ansonsten ungeladenen Polymersystemen einen viskositätserhöhenden Salzeffekt zu generieren. So beschreibt Plank et al in DE 19930031 betainhaltige Terpolymere und deren viskositätssteigernde Wirkung bei Salzzugabe. Schulz et al. Beschreibt in US 5153289 eine Kombination von SPE mit NVP und einen deutliche Viskositätsanstieg bei Zugabe von Salz, wohingegen Copolymere von AMPS und NVP einen inversen Effekt zeigen. Eine weitere wichtige Anwendung des Betains wird von Vanderlaan et. al. in US 5311223 als Comonomer für die Herstellung von Kontaktlinsen beschrieben, wobei dem SPE ein verbessertes Wasserrückhaltevermögen zugeschrieben wird. Alle bisherigen Publikationen beschreiben die Umsetzung der Betaine mit ungeladenen Monomer-Bausteinen.

Überraschend konnte nun gefunden werden, daß Betaine, wie z.B. SPE sich problemlos mit dem anionischen AMPS copolymerisieren lassen. Die Kombination der Copolymere mit Wasser führt zu neuartigen Gelen mit überraschenden rheologischen Eigenschaften. So zeigen Gele auf Basis von AMPS/SPE Copolymeren im Vergleich zu Gelen auf Basis kommerziell erhältlichen AMPS/NVP Copolymeren, wie z.B. Aristoflex AVC (Clariant), eine signifikante Verbesserung der Scherstabilität. Scherstabilität ist für die Herstellung und Anwendung dieser Produkte ein wichtiges Kriterium. So können Gele beispielsweise schneller hergestellt werden infolge stärkerer Rührung. Auch spielt Scherstabilität eine wichtige Rolle bei dem Einsatz als Viskosität modifizierendes Polymer bei flüssigen Bohrspülmitteln.

Als erfindungsgemäß werden gemäß Patentanspruch 1 Copolymer-Strukturen angesehen, die folgende Kriterien (1) bis (3) verfüllen:
(1) Als essentielle Komponente ist in allen erfindungsgemäßen Copolymeren eine oder mehrere betainische Monomereinheiten der Struktureinheit (1) enthalten. Der Gewichtsanteil der Struktureinheit (1) beträgt zwischen 0,1 und 99,9 Gew.-%, bezogen auf das Copolymer. 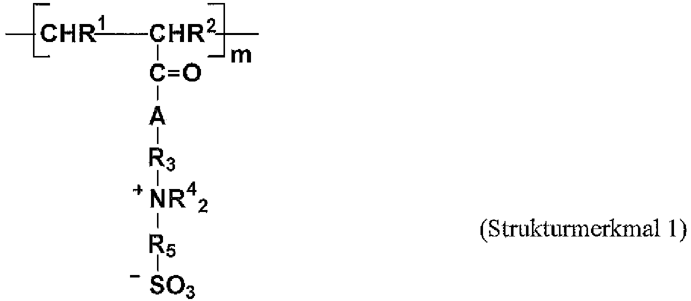 Darin gilt unabhängig voneinander:
   m = ganze Zahl ≥ 1;
   R¹ = H, C₁₋₃ Alkyl, -COOH;
   R²= H, C₁₋₃ Alkyl;
   R³ = n- oder iso-Alkylen der Formel -(CₓH₂ₓ)-, worin x ganzzahlig zwischen 1 und 30 ist;
   R⁴ = -H, Cycloalkyl, enthaltend vorzugsweise 3 bis 7 C-Atome, n- oder iso-Alkyl-, enhaltend vorzugsweise 1 bis 12 C-Atome, Aryl-, enthaltend vorzugsweise 5 bis 18 C-Atome,
   R⁵ = n- oder iso-Alkylen der Formel -(C_{y}H_{2y})-, worin y ganzzahlig zwischen 1 und 30 ist; A = -S-, -O-, -NR²-, worin R² = H, C₁₋₃ Alkyl.
(2) Weiterhin müssen als zweites essentielles Strukturmerkmal eine oder mehrere anionisch geladene Monomereinheiten mit einem Gewichtsanteil von 0,1 bis 99,9 Gew.-%, bezogen auf das Copolymer, vorhanden sein. 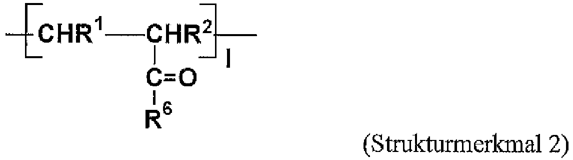 Darin gilt unabhängig voneinander:
   1 = ganze Zahl ≥ 1;
   R¹ = H, C₁₋₃ Alkyl, -COOH;
   R² = H, C₁₋₃ Alkyl;
   R⁶ = -OH, -OOH, -SH, -(CₓH₂ₓ)-COOH, -(CₓH₂ₓ)-SO₃H, -N(R²)-(CₓH₂ₓ)-SO₃H, worin - (CₓH₂ₓ)- = n- oder iso-Alkylen bedeutet und x ganzzahlig zwischen 1 und 30 ist.
(3) Zusätzlich finden ein oder mehrere nichtionische Comonomere Verwendung finden. Diese bilden die Comonomereinheiten des Strukturmerkmals 3 der erfindungsgemäßen Copolymere. Dabei beträgt der Gewichtsanteil der nichtionischen Comonomereinheiten 0 und 50 Gew.-%, bezogen auf das Copolymer. Es können sowohl mono- als auch di- oder polyvinylische Comonomere eingesetzt werden. Di- oder polyvinylische Comonomere führen dabei zur Vernetzung der Strukturen. Das Molekuargewicht der Comonomere beträgt vorzugsweise < 3 00 g/mol.

Zur Herstellung der in dieser Erfindung beschriebenen Copolymere wird bevorzugt die Technik der Fällungspolymerisation, besonders bevorzugt in einem tertiären Alkohol, verwendet.

Im Folgenden wird die Erfindung näher beschrieben. Als erfindungsgemäß werden in der vorliegenden Schrift polymere Grundkörper angesehen, die das Strukturmerkmal (1) beinhalten. Monomere dieser inneren Salze (auch Betaine genannt) wurden von der Firma Raschig (Ludwigshafen) kommerzialisiert und eignen sich ohne weitere Vorbehandlung zum Einsatz in der Polymerisation. Nachstehend ist eine nicht limitierende Liste von geeigneten Monomeren aufgezählt, die zur radikalischen Polymerisation eingesetzt werden können und das Strukturmerkmal (1) der erfindungs gemäßen Copolymere bilden. Einsetzbar sind prinzipiell alle Sulfobetaine, die einer radikalischen Polymerisation zugänglich sind. Bevorzugte Verwendung finden N,N-Dimethyl-N-(2-methacryloyloxyethyl)-N-(3-sulfopropyl)-ammoniumbetain (SPE), N,N-Dimethyl-N-(3-methacrylamidopropyl)-N-(3-sulfopropyl) ammoniumbetain (SPP) und 1-(3-Sulfopropyl)-2-vinylpyridiniumbetaine (SPV). Gemische von betainischen Spezies sind ebenfalls erfindungsgemäß. Der Gewichtsanteil des Strukturmerkmals (1) liegt zwischen 0,1 und 99,9 Gew.-%, vorzugsweise zwischen 0,1 und 50 Gew.-%, und besonders bevorzugt zwischen 3 und 25 Gew.-% der erfindungsgemäßen Copolymere.

Neben dem Strukturmerkmal (1) muss die polymere Basisstruktur ein weiteres Merkmal (2) aufweisen, das sich aus der Gruppe der anionischen, radikalisch polymerisierbaren Vinylverbindungen ableitet. Unter anionischen Monomeren im Sinne der Erfindung werden alle sauren Monomere verstanden, die in wässriger - oder organisch wässriger Umgebung einen pH-Wert < 7 ergeben. Folglich zählen zu dieser Gruppe alle von schwachen oder starken Mineralsäuren abgeleiteten Monomere wie Carbon-, Phosphon-, Phosphor- und Sulfonsäure mit einem oder mehreren leicht beweglichen (aziden) Protonen. Die erfindungsgemäßen Monomere können in freier Form oder in Form ihrer organischen oder anorganischen Salze (oder bei mehrwertigen Säuren auch Teilsalze) eingesetzt werden. Mischungen zweier oder mehrere Vertreter dieser Stoffgruppe schließt die Erfindung ebenfalls ein. Aufgrund ihrer besonders hohen Säurestärke wurden anionische Vertreter aus der Gruppe der vinylischen Sulfonsäuren als besonders geeignet identifiziert. Als Beispiele, die das Strukturmerkmal (2) illustrieren sollen, sind folgende Monomere zu benennen. (3-Sulfopropyl)-acrylat, Kaliumsalz (SPA); (3-Sulfopropyl)-methacrylat, Kaliumsalz (SPM), beide erhältlich von der Raschig GmbH, Ludwigshafen; 2-Acrylamidopropylmethylensulfonsäure (AMPS von Lubrizol) und dessen Salze, vornehmlich Alkali- und Ammoniumsalze und Vinylsulfonsäure-Na-Salz. Mischungen und/oder Mischsalze dieser Monomergruppe sind ebenfalls geeignet. Der Gewichtsanteil des Strukturmerkmals (2) liegt zwischen 0,1 und 99,9 Gew.-%, vorzugsweise zwischen 20 und 98 Gew.-%, besonders bevorzugt zwischen 50 und 80 Gew.-%.

Das Strukturmerkmal (3) umfasst alle nichtionischen Comonomereinheiten, die aus radikalisch polymerisierbaren Comonomeren mit einer oder mehreren vinylischen Funktionalitäten gebildet werden. Der Einsatz von Monomeren mit vinylischer Funktionalisierung ≥ 2 kann zur Vernetzung oder Verzweigung der entstehenden Polymerstrukturen führen. Beispiele für monofunktionalisierte Monomere sind Ester der (Meth)acrylsäure (z.B.: Hydroxyethylmethacrylat, Hydroxypropylmethacylat, Ethyl(meth)acrylat, Propylacrylat, Butylacrylat, Stearylacrylat, Laurylmethacrylat),. Neben dieser Auswahl an monofunktionalisierten Monomeren ist, wie eben erwähnt, auch der Einsatz von multifunktionalisierten Monomeren im Sinne der Erfindung, die zur Vernetzung und/oder Verzweigung der Polymerketten führe. Beispiele hierfür sind Methylenbisacrylamid, und Triacrylate bzw. Trimethacrylate (Tri-methylolpropantrimethacrylat, Trimethylolpropantriacrylat, 1,2,3 Glycerintrimethacrylat, 1,2,3-Glycerintriacrylat), wobei die letztere Gruppe von vernetzend wirkenden Verbindungen als bevorzugt erwähnt werden soll. Der Gewichtsanteil des Strukturmerkmals (3) liegt zwischen 0 und 50 Gew.-%, vorzugsweise zwischen 0,1 und 50 Gew.-% und besonders bevorzugt zwischen 0,2 und 25 Gew.-%, bezogen auf das Copolymer.

Gemäß Patentanspruch 1 lauten die erfindungsgemäßen Kombinationen der Strukturmerkmale 1-3 in den erfindungsgemäßen Copolymeren wie folgt:
(3-Sulfopropyl)-acrylat Kaliumsalz (SPA) (Strukturmerkmal 2), N,N-Dimethyl-N-(2-methacryloyloxyethyl)-N-(3-sulfopropyl)-ammoniumbetain (SPE) (Strukturmerkmal 1), und Trimethylolpropantrimethacrylat (Strukturmerkmal 3);
N,N-Dimethyl-N-(3-methacrylamidopropyl)-N-(3-sulfopropyl)-ammoniumbetain (SPP) (Strukturmerkmal 1), (3-Sulfopropyl)-methacrylat Kaliumsalz (SPM) (Strukturmerkmal 2), und Trimethylolpropantriacrylat (Strukturmerkmal 3);
N,N-Dimethyl-N-(3-methacrylamidopropyl)-N-(3-sulfopropyl)-ammoniumbetain (SPP) (Strukturmerkmal 1), (3-Sulfopropyl)-acrylat Kaliumsalz (SPA) und (3-Sulfopropyl)-methacrylat Kaliumsalz (SPM) (Strukturmerkmal 2), und Trimethylolpropantriacrylat (Strukturmerkmal 3);
N,N-Dimethyl-N-(3-methacrylamidopropyl)-N-(3-sulfopropyl)-ammoniumbetain (SPP) (Strukturmerkmal 1), 2-Acrylamido-2-propylmethylensulfonsäure, Ammonium-Salz (Strukturmerkmal 2), und
Trimethylolpropantrimethacrylat;
N,N-Dimethyl-N-(2-methacryloyloxyethyl)-N-(3-sulfopropyl)-ammoniumbetain (SPE) (Strukturmerkmal 1), 2-Acrylamido-2-propylmethylensulfonsäure, Ammonium-Salz (Strukturmerkmal 2), und Trimethylolpropantrimethacrylat (Strukturmerkmal 3);
N,N-Dimethyl-N-(2-methacryloyloxyethyl)-N-(3-sulfopropyl)-ammoniumbetain (SPE) (Strukturmerkmal 1), 2-Acrylamido-2-propylmethylensulfonsäure, Ammonium-Salz (Strukturmerkmal 2), und
Trimethylolpropantrimethacrylat und Hydroxyethylmethacrylat (HEMA) (Strukturmerkmal 3);
N,N-Dimethyl-N-(2-methacryloyloxyethyl)-N-(3-sulfopropyl)-ammoniumbetain (SPE) (Strukturmerkmal
1), (3-Sulfopropyl)-methacrylat Kaliumsalz (SPM) (Strukturmerkmal 2), und Stearylacrylat (Strukturmerkmal 3);
1-(3-Sulfopropyl)-2-vinylpyridiniumbetaine (SPV) (Strukturmerkmal 1), 2-Acrylamido-2-propylmethylnsulfonsäure, Natrium-Salz (Strukturmerkmal 2), und Methylenbisacrylamid sowie Hydroxyethylmethacrylat (HEMA) (Strukturmerkmal 3).

Alle in dieser Erfindung erwähnten Polymere wurden nach dem Fällungsverfahren hergestellt. Als Hauptmerkmale dieses Verfahrens ist zum einen eine wenigstens geringfügige Löslichkeit der Monomere im verwendeten Lösungsmittel vorauszusetzen und zum anderen eine nahezu vollständige Unlöslichkeit oder lediglich Quellbarkeit der entstehenden Polymere bei Überschreitung eines systemspezifischen mittleren Molekulargewichts im jeweiligen Lösungsmittel oder Lösungsmittelgemisch. In den Beispielen wird das Verfahren näher beschrieben. Zum Erzielen hoher mittlerer Molekulargewichte ist die Verwendung von Lösungsmitteln erforderlich, die sich gegenüber der eigentlichen Polymerisationsreaktion weitestgehend inert verhalten. Derartige Medien sind beispielsweise tertiäre Alkohole, insbesondere t-Butanol, welches sich aufgrund seines moderaten Siedepunktes in technischer Hinsicht als besonders geeignet erwies. Lösungsmittelgemische (z.B.: t-Butanol/Wasser) sind ebenfalls im Sinne der Erfindung.

Zur Auslösung der radikalischen Reaktion können neben stofflichen Initiatoren, wie zum Beispiel Azoisobutyronitril, Dilauroylperoxid, Benzoylperoxid, Wasserstoffperoxid, Kaliumperoxodisulfat, Natriumperoxodisulfat auch durch elektromagnetische Strahlung (insbes. UV-Strahlung), thermische Initiatorsysteme Anwendung finden. Unter toxikologischen Gesichtspunkten nehmen aliphatische Peroxoinitiatoren eine ausgezeichnete Stellung ein. Insbesondere geeignet und allgemein akzeptiert ist Dilauroylperoxid.

Im Falle von linearen, vernetzten oder verzweigten Makromoleküle kann die Bestimmung des hydrodynamischen Volumens durch dynamische Lichtstreuung herangezogen werden. Da Makromoleküle in Lösung selten eine Kugel, sondern vielmehr knäuelartige Strukturen oder Ellipsoide bilden, bezieht sich das hydrodynamische Volumen auf das Volumen einer reibungsäquivalenten Kugel. Im Sinne der Erfindung sind einzelne polymere Partikel, die im getrockneten Zustand von 1 nm bis 10 mm groß sind, bevorzugt ist der Bereich von 10 nm bis 1 mm, besonders bevorzugt ist der Bereich von 50 nm bis 50 µm. Die Bildung physikalischer Aggregate bestehend aus mehreren dieser Partikel führt nicht zur Verschlechterung der Eigenschaften der erfindungsgemäßen Produkte. Die Angabe eines bevorzugten Molekulargewichtsbereiches für vernetzte Polymere wird üblicherweise nicht vorgenommen, da vernetzten Systemen per Definition ein nahezu unendliches Molekulargewicht zugeordnet wird und damit eine Bevorzugung eines Teilbereiches keinen Sinn macht. Vernetzte Polyelektrolytsysteme, wie beispielsweise die Carbonsäure-basierenden Carbomere, sind in reinem Wasser hervorragende Verdicker und werden in vielen Anwendungsgebieten als "rheological modifier" eingesetzt. Die Scherstabilität der Gele variiert jedoch deutlich. Zur Messung der Scherstabilität wurde folgendes Messverfahren entwickelt.

Die synthetisierten Gelbildner werden jeweils mit Wasser zu einer 1 Gew.% igen Lösung gequollen, welche 4 h mit einem KPG-Rührer durchmischt werden, bis eine klare, homogene Lösung entstanden ist. Die so hergestellten Hydrogele werden mittels rheologischer Untersuchungen charakterisiert.

Die Viskositätsmessungen wurden mit einem schub-spannungskontrollierten Messsystem von Thermo Scientific (Haake Mars Rheometer) durchgeführt. Die gewählte Geometrie war eine Platte-Platte Messeinrichtung (Platte PP35 Ti/Meas Platte MP35 18/8, Kegeldurchmesser 35 mm). Der Abstand der Platten zwischen denen das Probenmaterial aufgetragen wurde betrug stets 0,5 mm. Die Gele wurden zwischen Platte und Platte eingebracht und bei Scherraten von 0,001 s⁻¹ bis 1.000 s⁻¹ vermessen. Die Proben wurden sechsmal in Folge vermessen, wobei zwischen den einzelnen Messungen jeweils Pausen von 5 Sekunden, einer Stunde, zwei Stunden, drei Stunden, vier Stunden und fünf Stunden zur Relaxation des Probenmaterials gemacht wurden. Die Probentemperatur wurde dabei konstant bei 25°C gehalten. Der pH-Wert der Lösungen war ggf. unter Verwendung geringer Mengen von Milchsäure auf 5 eingestellt.

Durch den Vergleich der Viskositäten vor und nach der Scherung wird der Viskositätsabfall - Index bestimmt.
Die nachfolgende Tabelle zeigt eindrucksvoll den Effekt des Betains auf die Scherstabilität.

**Tab. 1: Gemessene Viskositäten bei einer Scherrate von 100 s⁻¹**

| | **5 sek.** | **1 h** | **2 h** | **3 h** | **4 h** | **5 h** |
|---|---|---|---|---|---|---|
| **Polymer gemäß Beispiel (1)** | 3,7 | 3,05 | 2,37 | 1,77 | 1,23 | 0,77 |
| **Polymer gemäß Beispiel (2)** | 3,0 | 2,85 | 2,03 | 1,61 | 1,12 | 0,73 |
| **Polymer gemäß Beispiel (3)** | 3,60 | 3,03 | 2,33 | 1,70 | 1,21 | 0,76 |
| **Aristoflex AVC)** | 2,59 | 2,07 | 1,68 | 1,33 | 1,01 | 0,70 |

Kommentar: Die erfindungsgemäßen Beispiele 1,2 und 3 zeigen eindeutig eine verbesserte Scherstabilität gegenüber dem Handelsprodukt Aristoflex AVC.

Weitere Synthese-Beispiele zur Illustration der Erfindung werden im Folgenden konkretisiert. Hinweise für die praktische Durchführung der Polymerisationsreaktionen finden sich in "Praktikum der Makromolekularen Stoffe" Autoren H. Cherdron et al., Wiley VCH (1999):

### Herstellbeispiele:

### Beispiel (1) (erfindungsgemäß)

In einem 0,5 1 bzw. 1,0 1 Mehrhalskolben ausgestattet mit KPG-Rührer, Rückflusskühler, pH-Meter und Einleitung für NH₃ bzw. N₂ werden 245 g tert.-Butanol und 6,25 g Wasser vorgelegt. Dann werden 31,60 g 2-Acrylamido-2-methylpropansulfonsäure (fein gemörsert) eingetragen und dispergiert. Nun wird innerhalb von 30 min Ammoniak eingeleitet bis sich ein pH-Wert von 6-7 einstellt (Kontrolle über pH-Meter). Dabei verschwindet die ursprünglich vorhandene Dispersion und nur eine leichte Trübung verbleibt. Nun wird weitere 30 min gerührt. Das pH-Meter wird durch einen Thermometer ersetzt. Zu der trüben Lösung wird dann im Stickstoff-Gegenstrom 15 g SPE und 0,94 g Vernetzer (TMPTA) zugegeben. Das Reaktionsgemisch wird auf 60 °C erhitzt und die entsprechende Menge Katalysator, Dilauroylperoxid, zudosiert. Sobald sich ein weißer Niederschlag bildet und die Temperatur steigt, was den Beginn der Reaktion anzeigt, wird die Rührgeschwindigkeit von 300 U/min auf 100 U/min reduziert. Nach weiteren 15 min wird der Stickstoffstrom abgestellt. Nach Initiatorzugabe wird insgesamt 60 min bei 60 °C gerührt. Dann wird zum Rückfluss erhitzt und weitere 2 h gerührt bevor die Reaktionslösung auf Raumtemperatur abgekühlt wird. Das Lösungsmittel wird unter vermindertem Druck, 130 mbar und 40 °C Badtemperatur entfernt und anschließend bei 70 °C getrocknet. Der verbleibende weiße Rückstand wird bei 70 °C im Hochvakuumtrockenschrank 24 h getrocknet.

### Beispiel (2) (erfindungsgemäß)

**Verfahrensweise analog Beispiel (1)**

| | | |
|---|---|---|
| Monomereinsatz: | Strukturmerkmal (1) | 1,80 g *N*-(3-Sulfopropyl)-*N*-methacryloxyethyl-*N*,*N*-dimethylammonium-betain (SPE) |
| | Strukturmerkmal (2) | 15,80 g 2-Acrylamido-2-methylpropansulfonsäure (AMPS) |
| | Strukturmerkmal (3) | 0,36 g Trimethylolpropantriacrylat (TMPTA) |

### Beispiel (3) (Referenzbeispiel)

**Verfahrensweise analog Beispiel (1)**

| | | |
|---|---|---|
| Monomereinsatz: | 31,60 g (0,15 mol) | 2-Acrylamido-2-methylpropansufonsäure (AMPS) |
| | 13,92 g (0,05 mol) | *N*-(3-Sulfopropyl)-*N*-methacrylamidoethyl-*N*,*N*-dimethylammonium-betain |
| | 0,91 g (0,003mol) | Trimethylolpropantriacrylat (TMPTA) |

### Verwendungsbeispiele:

Das interessante Eigenschaftsprofil der erfindungsgemäßen Polymere legt Anwendungen im Bereich Kosmetik, aber auch in der Pharmazie nahe. Der Trend hin zu wässrigen Formulierungen und weg von schweren Cremes auf Ölbasis erfordert die Entwicklung neuer Verdickersysteme, die den Anforderungen heutiger Formulierungen, mit ihrer teilweise mehr als 30 Komponenten umfassenden Bestandteilsliste, gerecht wird. Im Vorangegangenen wurde bereits die außergewöhnliche Scherstabilität der in dieser Erfindung beschriebenen Polymer-Gele hervorgehoben. Dieses Profil macht sie geeignet für die unterschiedlichsten Anwendungen, in Kosmetik, Pharmazie und anderen Anwendungen geeignet, bei denen die Viskosität wässriger Formulierungen eingestellt werden muß.

**Saure Creme:**

| | | |
|---|---|---|
| Phase A | Methyl Glucose Sesquistearat | 2% |
| | Stearylalkohol | 2% |
| | Cyclomethicon | 10,5% |
| | Isoparaffin | 7,2% |
| | Capryloyl Salicylsäure | 1,4% |
| | Octyldodecanol | 5% |
| | Parfum | 0,3% |
| | | |
| Phase B | Wasser | q.s.p. 100% |
| | Meersalz | 0,10% |
| | Konservierungsstoffe | 0,5% |
| | Glycerin | 5% |
| | | |
| Phase C | Polymer gemäß Beispiel 1 | 2% |

Herstellung: In Phase B wird C durch Rühren eingetragen und dann nach Phase A eingerührt. Anschließend wird homogenisiert

Besonders geeignet erwies sich die Verwendung der beschriebenen Polymere bei der Herstellung transparenter, klarer Gele.

**Klares Gel:**

| | | |
|---|---|---|
| Phase A | Ethanol p.a. | 20 % |
| | Glycerin | 6 % |
| | Parfum | 0,30 % |
| | | |
| Phase B | Konservierungsmittel | 0,5 % |
| | Polymer gemäß Beispiel 3 | 1,0 % |
| | Wasser | q.s.p. 100 % |

Herstellung: In Phase B wird A durch Rühren eingetragen und homogenisiert

**Feuchtigkeitscreme**

| | | |
|---|---|---|
| Phase A | Glycerin | 12 % |
| | Isopropylpalmitat | 4,0 % |
| | Glyceryl-Stearat | 5,0 % |
| | Aloe Vera | 0,5 % |
| | Jojoba Öl | 2,0 % |
| | Parfum | 0,25 % |
| | | |
| Phase B | Konservierungsmittel | 0,5 % |
| | Polymer gemäß Beispiel 2 | 2,0 % |
| | Wasser | q.s.p. 100 % |

Herstellung: A wird in B eingerührt und homogenisiert.

Als Beispiel einer pharmazeutischen Anwendung soll die Herstellung eines Ultraschallgels dienen.

**Ultraschallgel**

| | |
|---|---|
| Glycerin | 15 % |
| Polymer gemäß Beispiel 1 | 1,5 % |
| Wasser | q.s.p. 100% |

Als Beispiele für den Bereich Wasch- und Reinigungsmittel dienen folgende Formulierungen:

**Saure Reiniger**

| | |
|---|---|
| Marlon PS 65 (Sasol) | 7% |
| Laurylethoxylat 7 | 2,5% |
| Citric acid | 14% |
| NaCl | 0,25% |
| Polymer gemäß Beispiel 1 | 1,2 % |
| Wasser | q.s.p. 100 % |

**Flüssiges Toilettengel,**

| | |
|---|---|
| Laurylsulfat | 2,5 % |
| Oleylethoxylat 7 | 5 % |
| Wasserstoffperoxid | 5 % |
| Citrate/Citric Acid | 8 % |
| Polymer gemäß Beispiel 2 | 1,3% |
| Wasser | q.s.p. 100% |

Die erfindungsgemäßen Polymere können auch zur Verbesserung von Baustoffgemischen eingesetzt werden. Dabei wird die Abbindezeit des Mörtelgemisches soweit verzögert, dass beispielsweise eine verlängerte Korrekturzeit beim Verlegen von Fliesen verbleibt:

**Der Fliesenkleber wird aus:**

| | |
|---|---|
| Portland-Zement PZ 45 | 30% |
| Quarzsand (< 0,5 mm) | 45% |
| Tylose MH 200 YP2 (Handelsprodukt der Fa. Shin-Etsu) | 4,0% |
| Polymer gem. Beispiel 1 | 0,2% |
| Wasser | q.s.p. 100% |

hergestellt. Denkbar sind auch Anwendungen in silikat- oder tonhaltigen Systemen z.B. als Abbindeverzögerer in Zement, bei der Förderung von Erdöl insbes. als Bohrspülung oder zur Aufarbeitung von kontaminierten Böden. Verdicker für Druckpasten und -tinten, Lacke oder Beschichtungsmassen im Allgemeinen sind häufig anfällig für Scherstress und könnten so verbessert werden.

## Patentansprüche

1. Copolymere enthaltend
a) als Strukturmerkmal 1 mindestens eine oder mehrere betainische Monomereinheit(en) gemäß der nachstehenden Formel mit einem Gewichtsanteil von 0,1 und 99,9 Gew.-%, bezogen auf das Copolymer 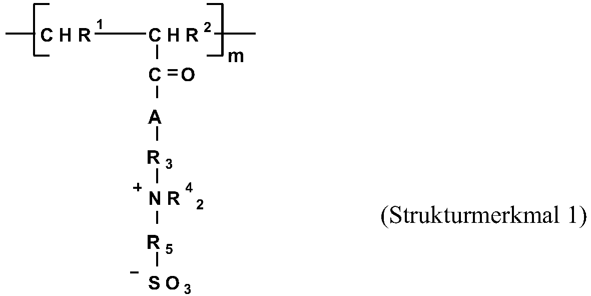 worin unabhängig voneinander gilt:
m = ganze Zahl ≥ 1;
R¹ = H, C₁₋₃ Alkyl, -COOH;
R² = H, C₁₋₃ Alkyl;
R³ = n- oder iso-Alkylen der Formel -(CₓH₂ₓ)-, worin x ganzzahlig zwischen 1 und 30 ist;
R⁴ = -H, Cycloalkyl, n- oder iso-Alkyl-, Aryl-;
R⁵ = n- oder iso-Alkylen der Formel -(C_{y}H_{2y})-, worin y ganzzahlig zwischen 1 und 30 ist;
A = -S-, -O-, -NR²-, worin R² = H, C₁₋₃ Alkyl;
b) als Strukturmerkmal 2 mindestens eine oder mehrere anionisch geladene Monomereinheit(en) gemäß der nachstehenden Formel mit einem Gewichtsanteil von 0,1 bis 99,9 Gew.-%, bezogen auf das Copolymer 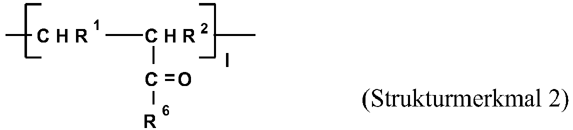 worin unabhängig voneinander gilt:
1 = ganze Zahl ≥ 1;
R¹ = H, C₁₋₃ Alkyl, -COOH;
R² = H, C₁₋₃ Alkyl;
R⁶ = -OH, -OOH, -SH, -(CₓH₂ₓ)-COOH, -(CₓH₂ₓ)-SO₃H, -N(R²)-(CₓH₂ₓ)-SO₃H, worin-(CₓH₂ₓ)- = n- oder iso-Alkylen bedeutet und x ganzzahlig zwischen 1 und 30 ist;
c) als Strukturmerkmal 3 eine oder mehrere nichtionische Comonomereinheit(en) mit einem Gewichtsanteil von 0 und 50 Gew.-%, bezogen auf das Copolymer,
wobei die Kombinationen der Strukturmerkmale 1-3 lauten: (3-Sulfopropyl)-acrylat Kaliumsalz (SPA) (Strukturmerkmal 2), N,N-Dimethyl-N-(2-methacryloyloxyethyl)-N-(3-sulfopropyl)-ammoniumbetain (SPE) (Strukturmerkmal 1), und Trimethylolpropantrimethacrylat (Strukturmerkmal 3);
N,N-Dimethyl-N-(3-methacrylamidopropyl)-N-(3-sulfopropyl)-ammoniumbetain (SPP) (Strukturmerkmal 1), (3-Sulfopropyl)-methacrylat Kaliumsalz (SPM) (Strukturmerkmal 2), und Trimethylolpropantriacrylat (Strukturmerkmal 3);
N,N-Dimethyl-N-(3-methacrylamidopropyl)-N-(3-sulfopropyl)-ammoniumbetain (SPP) (Strukturmerkmal 1), (3-Sulfopropyl)-acrylat Kaliumsalz (SPA) und (3-Sulfopropyl)-methacrylat Kaliumsalz (SPM) (Strukturmerkmal 2), und Trimethylolpropantriacrylat (Strukturmerkmal 3);
N,N-Dimethyl-N-(3-methacrylamidopropyl)-N-(3-sulfopropyl)-ammoniumbetain (SPP) (Strukturmerkmal 1), 2-Acrylamido-2-propylmethylensulfonsäure, Ammonium-Salz (Strukturmerkmal 2), und Trimethylolpropantrimethacrylat;
N,N-Dimethyl-N-(2-methacryloyloxyethyl)-N-(3-sulfopropyl)-ammoniumbetain (SPE) (Strukturmerkmal 1), 2-Acrylamido-2-propylmethylensulfonsäure, Ammonium-Salz (Strukturmerkmal 2), und Trimethylolpropantrimethacrylat (Strukturmerkmal 3);
N,N-Dimethyl-N-(2-methacryloyloxyethyl)-N-(3-sulfopropyl)-ammoniumbetain (SPE) (Strukturmerkmal 1), 2-Acrylamido-2-propylmethylensulfonsäure, Ammonium-Salz (Strukturmerkmal 2), und Trimethylolpropantrimethacrylat und Hydroxyethylmethacrylat (HEMA) (Strukturmerkmal 3);
N,N-Dimethyl-N-(2-methacryloyloxyethyl)-N-(3-sulfopropyl)-ammoniumbetain (SPE) (Strukturmerkmal 1), (3-Sulfopropyl)-methacrylat Kaliumsalz (SPM) (Strukturmerkmal 2), und Stearylacrylat (Strukturmerkmal 3);
1-(3-Sulfopropyl)-2-vinylpyridiniumbetaine (SPV) (Strukturmerkmal 1), 2-Acrylamido-2-propylmethylensulfonsäure, Natrium-Salz (Strukturmerkmal 2), und Methylenbisacrylamid sowie Hydroxyethylmethacrylat (HEMA) (Strukturmerkmal 3).

2. Copolymere gemäß Anspruch 1, enthaltend 0,1 bis 50 Gew.-% des Strukturmerkmals 1, 20 bis 98 Gew.-% des Strukturmerkmals 2 und 0,1 bis 50 Gew.-% des Strukturmerkmals 3, jeweils bezogen auf das Copolymer.

3. Copolymere gemäß Anspruch 1, enthaltend 3 bis 25 Gew.-% des Strukturmerkmals 1, 50 bis 80 Gew.-% des Strukturmerkmals 2 und 0,2 bis 25 Gew.-% des Strukturmerkmals 3, jeweils bezogen auf das Copolymer.

4. Copolymere gemäß einem der Ansprüche 1-3, enthaltend eine vernetzte Struktur, welche durch Verwendung ein oder mehrerer nichtionischer Comonomere mit mindestens zwei vinylischen polymerisationsaktiven Gruppen, die das Strukturmerkmal 3 bilden, erhältlich ist.

5. Verwendung von Copolymeren gemäß einem der Ansprüche 1-4 in kosmetischen oder pharmazeutischen Zubereitungen oder als viskositätsmodifizierendes Copolymer bei flüssigen Bohrspülmitteln.

6. Verwendung von Copolymeren gemäß einem der Ansprüche 1-4 als Verdicker.

7. Verwendung von Copolymeren gemäß einem der Ansprüche 1-4 als Verdicker elektrolythaltiger Lösungen mit einem Elektrolytgehalt größer als 0,1 Gew.% bezogen auf die Gesamtformulierung.

8. Verwendung von Copolymeren gemäß einem der Ansprüche 1-4 in wässrigen Formulierungen mit einem pH-Wert < 6,0.

9. Verwendung von Copolymeren gemäß einem der Ansprüche 1-4 in Wasch- und Reinigungsmitteln.

10. Verwendung von Copolymeren gemäß einem der Ansprüche 1-4 als Verdicker für Systeme mit Gehalten an mit Wasser mischbaren organischen Lösungsmitteln von 20-99 Gew.%.

11. Verfahren zur Herstellung von Copolymeren, wie sie in einem der Ansprüche 1-4 definiert sind, welches eine Fällungspolymerisation, vorzugsweise in einem tertiären Alkohol, umfasst.

## Claims

1. Copolymers comprising
a) as structural feature 1 at least one betainic monomer unit according to the formula below with a proportion by weight of from 0.1 to 99.9% by weight, based on the copolymer 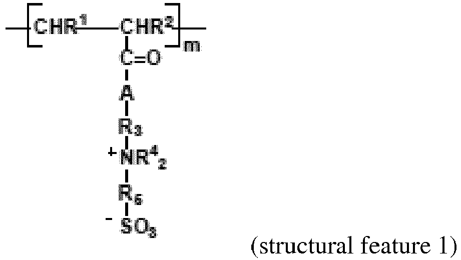 in which the mutually independent definitions are as follows:
m = integer ≥ 1;
R¹ = H, C₁₋₃ alkyl, -COOH;
R² = H, C₁₋₃ alkyl;
R³ = n- or isoalkylene of the formula -(CₓH₂ₓ)-, in which x is an integer from 1 to 30;
R⁴ = -H, cycloalkyl, n- or isoalkyl, aryl;
R⁵ = n- or isoalkylene of the formula -(C_{y}H_{2y})-, in which y is an integer from 1 to 30;
A = -S-, -O-, -NR², in which R² = H, C₁₋₃ alkyl;
b) as structural feature 2 at least one anionically charged monomer unit according to the following formula with a proportion by weight of from 0.1 to 99.9% by weight, based on the copolymer 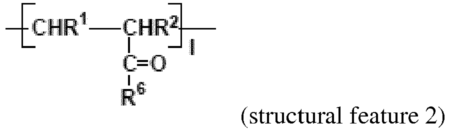 in which the mutually independent definitions are:
1 = integer ≥ 1;
R¹ = H, C₁₋₃ alkyl, -COOH;
R² = H, C₁₋₃ alkyl;
R⁶ = -OH, -OOH, -SH, -(CₓH₂ₓ)-COOH, -(CₓH₂ₓ)-SO₃H, -N(R²)-(CₓH₂ₓ)-SO₃H, in which -(CₓH₂ₓ)- = n- or isoalkylene and x is an integer from 1 to 30;
c) as structural feature 3 one or more non-ionic comonomer units with a proportion by weight of from 0 to 50% by weight, based on the copolymer, wherein the combinations of structural features 1 to 3 are:
(3-sulphopropyl)acrylate potassium salt (SPA) (structural feature 2), N,N-dimethyl-N-(2-methacryloyloxyethyl)-N-(3-sulphopropyl)ammonium betaine (SPE) (structural feature 1), and trimethylolpropane trimethacrylate (structural feature 3);
N,N-dimethyl-N- (3-methacrylamidopropyl)-N-(3-sulphopropyl)ammonium betaine (SPP) (structural feature 1), (3-sulphopropyl)methacrylate potassium salt (SPM) (structural feature 2), and trimethylolpropane triacrylate (structural feature 3);
N,N-dimethyl-N- (3-methacrylamidopropyl)-N-(3-sulphopropyl)ammonium betaine (SPP) (structural feature 1), (3-sulphopropyl)acrylate potassium salt (SPA) and (3-sulphopropyl)methacrylate potassium salt (SPM) (structural feature 2), and trimethylolpropane triacrylate (structural feature 3);
N,N-dimethyl-N- (3-methacrylamidopropyl)-N-(3-sulphopropyl)ammonium betaine (SPP) (structural feature 1), 2-acrylamido-2-propylmethylenesulphonic acid, ammonium salt (structural feature 2), and trimethylolpropane trimethacrylate;
N,N-dimethyl-N-(2-methacryloyloxyethyl)-N-(3-sulphopropyl)ammonium betaine (SPE) (structural feature 1), 2-acrylamido-2-propylmethylenesulphonic acid, ammonium salt (structural feature 2), and trimethylolpropane trimethacrylate (structural feature 3);
N,N-dimethyl-N-(2-methacryloyloxyethyl)-N-(3-sulphopropyl)ammonium betaine (SPE) (structural feature 1), 2-acrylamido-2-propylmethylenesulphonic acid, ammonium salt (structural feature 2), and trimethylolpropane trimethacrylate and hydroxyethyl methacrylate (HEMA) (structural feature 3);
N,N-dimethyl-N-(2-methacryloyloxyethyl)-N-(3-sulphopropyl)ammonium betaine (SPE) (structural feature 1), (3-sulphopropyl)methacrylate potassium salt (SPM) (structural feature 2), and stearyl acrylate (structural feature 3);
1-(3-sulphopropyl)-2-vinylpyridinium betaine (SPV) (structural feature 1), 2-acrylamido-2-propylmethylsulphonic acid, sodium salt (structural feature 2), and methylenebisacrylamide, and also hydroxyethyl methacrylate (HEMA) (structural feature 3).

2. Copolymers according to Claim 1, comprising from 0.1 to 50% by weight of the structural feature 1, from 20 to 98% by weight of the structural feature 2 and from 0.1 to 50% by weight of the structural feature 3, based in each case on the copolymer.

3. Copolymers according to Claim 1, comprising from 3 to 25% by weight of the structural feature 1, from 50 to 80% by weight of the structural feature 2 and from 0.2 to 25% by weight of the structural feature 3, based in each case on the copolymer.

4. Copolymers according to any one of claims 1 to 3, comprising a crosslinked structure which is obtainable via use of one or more non-ionic comonomers having at least two vinylic groups which have polymerization activity, where these form the structural feature 3.

5. Use of copolymers according to any one of claims 1 to 4 in cosmetic or pharmaceutical preparations or as viscosity-modifying copolymer in liquid drilling fluids.

6. Use of copolymers according to any one of claims 1 to 4 as thickeners.

7. Use of copolymers according to any one of claims 1 to 4 as thickeners of electrolyte-containing solutions with electrolyte content greater than 0.1% by weight, based on the entire formulation.

8. Use of copolymers according to any one of claims 1 to 4 in aqueous formulations with pH < 6.0.

9. Use of copolymers according to any one of claims 1 to 4 in detergents and cleaning compositions.

10. Use of copolymers according to any one of claims 1 to 4 as thickeners for systems with from 20-99% by weight content of water-miscible organic solvents.

11. Process which can produce copolymers as defined in any one of claims 1 to 4 and which encompasses precipitation polymerization, preferably in a tertiary alcohol.

## Revendications

1. Copolymères comprenant:
a) en tant que caractéristique structurelle 1 au moins une ou plusieurs unité(s) de monomères bétaïnique(s) selon la formule ci-après ayant une proportion en poids de 0,1 à 99,9 % en poids, relative au copolymère 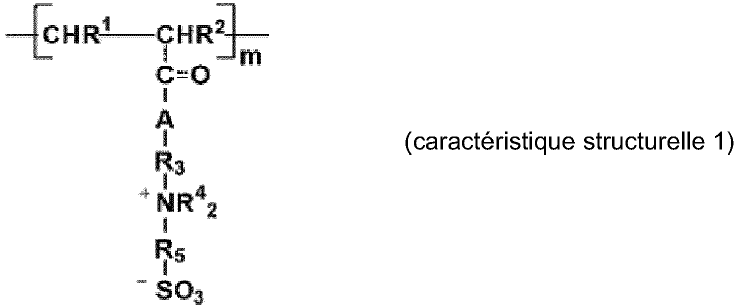 dans lequel vaut indépendamment l'un de l'autre:
m représente un nombre entier ≥ 1;
R¹ représente H, un groupe alkyle en C₁₋₃, -COOH;
R² représente H, un groupe alkyle en C₁₋₃;
R³ représente un groupe n- ou iso-alkylène ayant la formule -(CₓH₂ₓ)-, dans laquelle x est un nombre entier entre 1 et 30;
R⁴ représente -H, un groupe cycloalkyle, un groupe n- ou iso-alkyle, un groupe aryle-;
R⁵ représente un groupe n- ou iso-alkylène ayant la formule -(C_{y}H_{2y})-, dans laquelle y est un nombre entier entre 1 et 30;
A représente -S, -O-, -NR², dans lequel R² représente H, un groupe alkyle en C₁₋₃;
b) en tant que caractéristique structurelle 2 au moins une ou plusieurs unité(s) chargée(s) anioniquement selon la formule ci-après ayant une proportion en poids de 0,1 à 99,9 % en poids, relative au copolymère 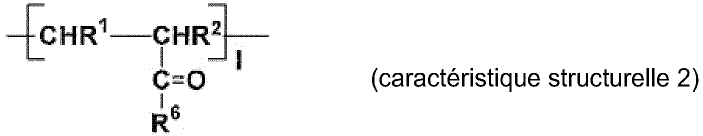 dans laquelle vaut indépendamment l'un de l'autre:
I représente un nombre entier ≥ 1;
R¹ représente H, un groupe alkyle en C₁₋₃, -COOH;
R² représente H, un groupe alkyle en C₁₋₃;
R⁶ représente -OH, -OOH, -SH, -(CₓH₂ₓ)-COOH, -(CₓH₂ₓ)-SO₃H, -N(R²)-(CₓH₂ₓ)-SO₃H, ou -(CₓH₂ₓ)- représente un groupe n- ou iso-alkylène et x est un nombre entier entre 1 et 30;
c) en tant que caractéristique structurelle 3 une ou plusieurs unité(s) de co-monomère(s) non-ionique(s) ayant une proportion en poids de 0 à 50 % en poids relative au copolymère,
les combinaisons de caractéristiques structurelles 1 à 3 étant telles: le sel de potassium (3-sulfopropyl)acrylate (SPA) (caractéristique structurelle 2), la N,N-diméthyl-N-(2-méthacryloyloxyéthyl)-N-(3-sulfopropyl)-ammoniumbétaïne (SPE) (caractéristique structurelle 1), et le triméthacrylate de triméthylolpropane (caractéristique structurelle 3);
la N,N-diméthyl-N-(3-méthacrylamidopropyl)-N-(3-sulfopropyl)-ammoniumbétaïne (SPP) (caractéristique structurelle 1), le sel de potassium (3-sulfopropyl)-méthacrylate (SPM) (caractéristique structurelle 2), et le triméthacrylate de triméthylolpropane (caractéristique structurelle 3);
la N,N-diméthyle-N-(3-méthacrylamidopropyl)-N-(3-sulfopropyl)-ammonium-bétaïne (SPP) (caractéristique structurelle 1), le sel de potassium (3-sulfopropyl)-acrylate (SPA) et le sel de potassium de (3-sulfopropyl)-méthacrylate (SPM) (caractéristique structurelle 2), et le triméthacrylate de triméthylolpropane (caractéristique structurelle 3);
la N,N-diméthyle-N-(3-méthacrylamidopropyl)-N-(3-sulfopropyl)-ammonium-bétaïne (SPP) (caractéristique structurelle 1), l'acide 2-acrylamido-2-propylméthylènesulfonique, sel d'ammonium (caractéristique structurelle 2), et le triméthacrylate de triméthylolpropane;
la N,N-diméthyl-N-(2-méthacryloyloxyéthyl)-N-(3-sulfopropyl)-ammoniumbétaïne (SPE) (caractéristique structurelle 1), l'acide 2-acrylamido-2-propylméthylène-sulfonique, sel d'ammonium (caractéristique structurelle 2), et le triméthacrylate de triméthylolpropane (caractéristique structurelle 3);
la N,N-diméthyl-N-(2-méthacryloyloxyéthyl)-N-(3-sulfopropyl)-ammoniumbétaïne (SPE) (caractéristique structurelle 1), l'acide 2-acrylamido-2-propylméthylène-sulfonique, sel d'ammonium (caractéristique structurelle 2), et le triméthacrylate de triméthylolpropane et le méthacrylate d'hydroxy éthyle (HEMA) (caractéristique structurelle 3);
la N,N-diméthyl-N-(2-méthacryloyloxyéthyl)-N-(3-sulfopropyl)-ammoniumbétaïne (SPE) (caractéristique structurelle 1), le sel de potassium (3-sulfopropyl)-méthacrylate (SPM) (caractéristique structurelle 2), et l'acrylate de stéaryle (caractéristique structurelle 3);
la 1-(3-sulfopropyl)-2-vinylpyridiniumbétaïne (SPV) (caractéristique structurelle 1), l'acide 2-acrylamido-2-propylméthylènesulfonique, sel de sodium (caractéristique structurelle 2), et la méthylènebisacrylamide ainsi que le méthacrylate d'hydroxy éthyle (HEMA) (caractéristique structurelle 3).

2. Copolymères selon la revendication 1, contenant 0,1 à 50 % en poids de la caractéristique structurelle 1, 20 à 98 % en poids de la caractéristique structurelle 2 et 0,1 à 50 % en poids de la caractéristique structurelle 3, respectivement relatif au copolymère.

3. Copolymères selon la revendication 1, contenant 3 à 25 % en poids de la caractéristique structurelle 1, 50 à 80 % en poids de la caractéristique structurelle 2 et 0,2 à 25 % en poids de la caractéristique structurelle 3, respectivement relatif au copolymère.

4. Copolymères selon selon l'une des revendications 1 à 3, contenant une structure réticulée, laquelle peut être obtenue par utilisation d'un ou de plusieurs comonomères non-ioniques avec au moins deux groupes vinyliques ayant une activité de polymérisation, qui forment la caractéristique structurelle 3.

5. Utilisation de copolymères selon l'une des revendications 1 à 4 en des préparations cosmétiques ou pharmaceutiques ou en tant que copolymère modifiant la viscosité en des fluides de forage.

6. Utilisation de copolymères selon l'une des revendications 1 à 4 en tant qu'agent d'épaississement.

7. Utilisation de copolymères selon l'une des revendications 1 à 4 en tant qu'agent d'épaississement de solutions contenant des électrolytes ayant une teneur en électrolytes supérieure à 0,1 % en poids relative à la formulation totale.

8. Utilisation de copolymères selon l'une des revendications 1 à 4 en des formulations aqueuses ayant une valeur pH < 6,0.

9. Utilisation de copolymères selon l'une des revendications 1 à 4 en des agents de lavage et de nettoyage.

10. Utilisation de copolymères selon l'une des revendications 1 à 4 en tant qu'agent d'épaississement pour des systèmes avec des teneurs de solvants organiques miscible avec de l'eau de 20 à 99 % en poids.

11. Procédé pour la préparation de copolymères, tels qu'ils sont définis dans une des revendications 1 à 4, lequel comprend une polymérisation par précipitation, préférablement dans un alcool tertiaire.
